# EUROPÄISCHE PATENTSCHRIFT

(11) **EP 0 511 224 B1**
(45) Veröffentlichungstag und Bekanntmachung des Hinweises auf die Patenterteilung: **19.11.1997**
(21) Anmeldenummer: 91900807.8
(22) Anmeldetag: 24.12.1990
(51) Int. Cl.: A41D 27/12

(54) **WIRKSTOFF-IMPRÄGNIERTES TUCH**
CLOTH IMPREGNATED WITH AN ACTIVE SUBSTANCE
CHIFFON IMPREGNE D'UNE MATIERE ACTIVE

(30) Priorität: 15.01.1990 DE 4000920; 08.03.1990 DE 4007275
(43) Veröffentlichungstag der Anmeldung: 04.11.1992
(73) Patentinhaber: TEBBE, Gerold, 98000 Monaco (MC)
(72) Erfinder: TEBBE, Gerold, 98000 Monaco (MC)
(74) Vertreter: Ostertag, Reinhard
(86) Internationale Anmeldenummer: EP9002303
(87) Internationale Veröffentlichungsnummer: WO9110375

(56) Entgegenhaltungen:
- EP-A- 0 317 658
- EP-A- 0 332 066
- DE-A- 2 004 872
- DE-A- 3 415 156
- DE-A- 3 447 833
- US-A- 3 623 659
- US-A- 4 338 686
- US-A- 4 514 461

## Beschreibung

Die Erfindung betrifft ein wirkstoff-imprägniertes Tuch gemäß dem Oberbegriff des Anspruches 1.

Ein derartiges Tuch ist in der DE 34 47 83 A1 beschrieben. Es wird dort vorgeschlagen, die unterschiedlichen Wirkstoffe wie Pflegemittel für Schuhe, abrasive Wirkstoffe und auch weitere Wirkstoffe wie Heilstoffe mikrozuverkapseln und diese Mikrokapseln auf ein Trägermaterial zu bringen. Nach Zerstörung dieser Mikrokapseln können die frei gewordenen Wirkstoffe dann mit einer Oberfläche in Kontakt treten, über welche das Wirkstoff-imprägnierte Tuch bewegt wird. Nachdem dieses bekannte Wirkstoff-imprägnierte Tuch für Kontaktberührung zwischen dem Wirkstoff und dem zu behandelnden Substrat ausgelegt ist, eignet es sich nicht für Zwecke der Inhalationstherapie.

In der WO 88/03765 ist ferner eine Schweißeinlage offenbart, welche in ein textiles Trägermaterial eingebettete Mikrokapseln aufweist. Diese enthalten Wirkstoffe, die einer Schweißbildung und Entzündungen entgegenwirken. Unter Verwendungsbedingungen befinden sich derartige Schweißeinlagen zwischen einem Kleidungsstück und der Haut des Benutzers, und die durch Zerstören der Mikrokapseln durch Körperwärme und Feuchtigkeit freigesetzten Wirkstoffe wirken in direktem Kontakt auf die Haut des Benutzers ein.

Durch die vorliegende Erfindung soll ein wirkstoff-imprägniertes Tuch gemäß dem Oberbegriff des Anspruches 1 so weitergebildet werden, daß es als medizinischer Schal zu auch rascher Abgabe des Wirkstoffes befähigt ist, trotzdem aber keine dichte Umhüllung für die Langzeitlagerung braucht.

Diese Aufgabe ist erfindungsgemäß gelöst durch ein Wirkstoff-imprägniertes Tuch gemäß Anspruch 1.

Bei dem erfindungsgemäßen Wirkstoff-imprägnierten Tuch erfolgt das Öffnen der leicht zerstörbaren Mikrokapseln durch Wärme und/oder Feuchtigkeitseinwirkung. Letzteres hat zugleich den Vorteil, daß der Wirkstoff nur dort freigegeben wird, wo ein Hautkontakt besteht, während die anderen Mikrokapseln geschlossen bleiben und bei einem späteren Anwenden des Tuches genauso leicht geöffnet werden können. Diese kontrollierte Freigabe des Wirkstoffes ist ein erheblicher Vorteil gegenüber flüssigkeitsgetränkten Tüchern, die z.B. bei Parfümierung einen sehr starken, benachbarte Personen störenden Geruch abgeben. Auch müssen diese Tücher nach der ersten Anwendung weggeworfen werden.

Die Erfindung schafft einen Schal, der einerseits eine medizinische Wirkung hat, andererseits ein ansprechendes Äußeres aufweist und ein Wegwerfartikel ist.

Der erfindungsgemäße Schal läßt sich lange lagern, bis er zum Einsatz kommt, da das Medikament in den Mikrokapseln zuverlässig zurückgehalten wird und erst unter Einfluß von Körperwärme und/oder Schweiß und/oder Druck oder Reibung freigegeben wird. Vliesstoffe auf Zellstoffbasis und gegebenenfalls Kunststoffzumischung sind als preiswerte Massenware auf dem Markt erhältlich und lassen sich auch verhältnismäßig einfach mit mikroverkapselten Medikamenten "füllen". Nach Verbrauch des Medikamentes kann der ganze Schal einfach weggeworfen werden. Darüber hinaus hat der Schal auch ein gutes Schluckvermögen für Schweiß.

Bei dem erfindungsgemäßen medizinischen Schal ist gewährleistet, daß der nach dem Öffnen der Mikrokapseln freigegebene Wirkstoff nicht von der Grundstruktur selbst aufgenommen wird, sondern an der Oberfläche des Tuches bereitgestellt wird.

Vorteilhafte Weiterbildungen der Erfindung sind in Unteransprüchen angegeben.

Die Weiterbildung der Erfindung gemäß Anspruch 2 ermöglicht es, bei insgesamt geringen Kosten einen äußerlich ansprechenden Schal zu haben, der wie ein üblicher dekorativer Schal längere Endabschnitte aufweist, die vom Hals des Benutzers nach unten hängen und geknüpft oder gefaltet werden können.

Gemäß Anspruch 3 erhält man einen Schal, bei welchem man ein flüchtiges Medikament gezielt in den Schalbereichen hat, die für eine mechanische Zerstörung der Mikrokapseln durch den Benutzer besonders leicht zugänglich sind und von denen aus aufsteigende Medikamentendämpfe besonders gut die Atemwege erreichen. Man kann so z.B. einen Schal mit einem Gegenmittel gegen Heuschnupfen realisieren, bei dem die Freigabe des Medikamentes durch den Benutzer rasch und einfach zu dem Zeitpunkt erfolgen kann, zu dem er sich in ein Wiesengebiet hineinbewegt.

Die Weiterbildung der Erfindung gemäß Anspruch 4 ist im Hinblick auf ein gutes Aufsaugen von Schweiß unter Trockenhaltung der Haut des Benutzers von Vorteil.

Mit der Weiterbildung der Erfindung gemäß Anspruch 5 wird erreicht, daß sich Feuchtigkeit und Wärme im Inneren des Schals stauen, was eine besonders wirksame Freigabe des mikroverkapselten Medikamentes gewährleistet.

Dabei ist die Weiterbildung gemäß Anspruch 6 sowohl im Hinblick auf ein besonders effektives Zurückhalten der Wärme als auch im Hinblick auf ein ansprechendes Äußeres des Schals von Vorteil.

Auch die Weiterbildung der Erfindung gemäß Anspruch 7 dient dazu, dem Schal ein ansprechendes Äußeres zu geben, so daß dieser nicht als medizinisches Mittel zum Zweck sondern zugleich als Schmuck empfunden wird.

Die Weiterbildungen der Erfindung gemäß den Ansprüchen 8 bis 11 und 13 geben Größenverteilungen und Wandstärkenverteilungen der Mikrokapseln vor, die sich im Hinblick auf eine rasche erste Wirkstofffreigabe und anschließend im wesentlichen konstant bleibende Wirkstofffreigabe besonders empfehlen.

Bei einem Tuch gemäß Anspruch 12 kann der Benutzer die Menge des freigegebenen Wirkstoffes über den auf das Tuch ausgeübten Druck dosieren.

Besteht das Wandmaterial der Mikrokapseln aus durch Wärme und/oder Feuchtigkeit zerstörbarem Material, so kann man bei einem Tuch gemäß Anspruch 12 über das Mischungsverhältnis der Mikrokapseln unterscheidlicher Wandstärke und/oder unterschiedlichen Durchmessers den zeitlichen Verlauf der Freigabe des Wirkstoffes steuern. Dabei gilt grob gesprochen, daß bei gegebenem Kapseldurchmesser die Stabilität der Kapseln mit wachsender Wandstärke zunimmt, während bei gegebener Wandstärke die Stablität der Kapseln mit wachsendem Durchmesser abnimmt. Durch geeignete Mischung von Mikrokapseln unterschiedlicher Wandstärke und/oder unterschiedlichen Durchmessers läßt sich somit insbesondere eine rasch auf einen konstanten Wert ansteigende und dann bis zur Erschöpfung des Wirkstoffvorrates im wesentlichen konstant bleibende Wirkstoffabgabe einstellen.

Die Weiterbildung der Erfindung gemäß Anspruch 14 dient einer besonders leichten mechanischen Zerstörbarkeit der Mikrokapseln.

Nachstehend wird die Erfindung anhand von Ausführungsbeispielen unter Bezugnahme auf die Zeichnung näher erläutert. In dieser zeigen:
- Figur 1:: einen stark vergrößerten Schnitt durch ein Taschentuch, welches mit mikroverkapseltem Öl imprägniert ist;
- Figur 2:: einen nochmals stark vergrößerten Schnitt durch eine der Mikrokapseln und die Verbindungsstelle zwischen dieser Mikrokapsel und der textilen Grundstruktur;
- Figur 3:: einen Schnitt durch eine abgewandelte Mikrokapsel mit Sollbruchstellen;
- Figur 4:: einen nochmals vergrößerten Schnitt durch einen Wandabschnitt einer weiter abgewandelten Mikrokapsel mit Sollbruchstellen;
- Figur 5:: eine Aufsicht auf einen mit einem Medikament imprägnierten Schal sowie auf einen Abschnitt einer Endlosbahn, aus welcher der Schal hergestellt ist;
- Figur 6:: einen senkrechten Schnitt durch einen Abschnitt des in Figur 5 gezeigten Schales in vergrößertem Maßstabe; und
- Figur 7:: eine ähnliche Ansicht wie Figur 5, in der ein abgewandelter Schal gezeigt ist.

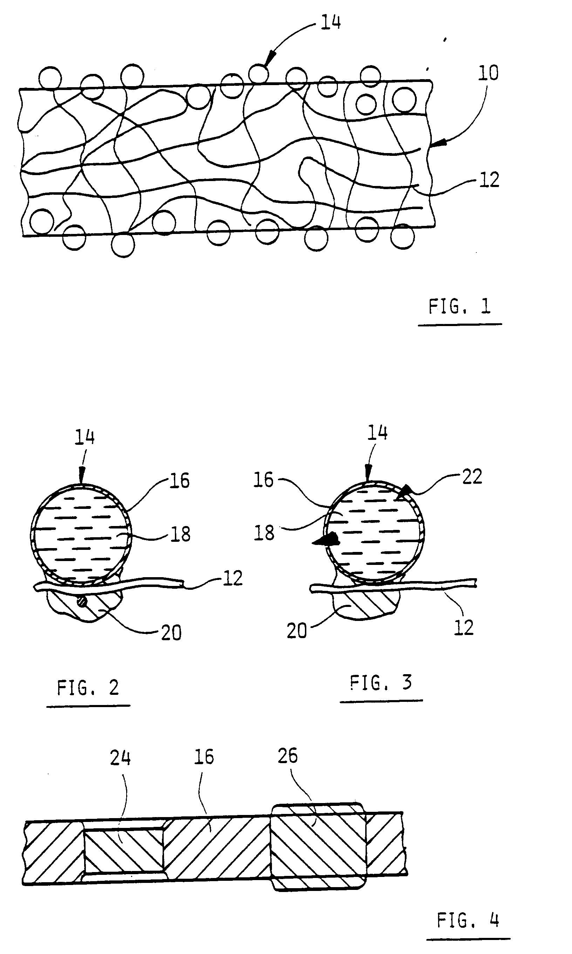

In Figur 1 ist eine vliesartige textile Grundstruktur eines Taschentuches insgesamt mit 10 bezeichnet. Sie besteht aus mehr oder weniger geordneten einzelnen Fasern 12.

Von den beiden Außenseiten her ist die Grundstruktur 10 mit Mikrokapseln 14 versehen. Dies kann z.B. dadurch erfolgen, daß man die Außenseite der Grundstruktur 10 mit einem Bindemittel überwalzt und die Mikrokapseln unter Verwendung eines Luftstromes oder mit einem elektrostatischen Sprühgerät gegen die Außenseiten der Grundstruktur 10 sprüht.

Die Mikrokapseln enthalten eine Pflegeöl, welches, falls gewünscht, zusätzlich mit Duftstoffen versehen sein kann. In der Praxis haben die Mikrokapseln einen Durchmesser von etwa 100 bis 500 µm und eine Wandstärke von 1 bis 5 µm. Die Kapseln habe also ein verglichen mit dem Wandvolumen großes Nutzvolumen; da die Wand dünn ist, lassen sich die Kapseln auch durch kleine mechanische Kräfte aufbrechen.

Zumindest die der Fasern 12, die der Außenseite der Grundstruktur 10 benachbart sind, bestehen aus einem solchen Material oder sind so ausgerüstet, daß sie das Pflegeöl nicht aufsaugen.

Wird das in Figur 1 gezeigte wirkstoff-imprägnierte Tuch auf der Haut gerieben, so werden die in Kontakt mit der Haut stehenden Mikrokapseln zerstört, und es wird Öl auf die Haut aufgetragen. Hierdurch wird ein Entzünden der Nase durch häufigen Gebrauch von Papiertaschentüchern verhindert.

Das in Figur 1 gezeigte wirkstoff-imprägnierte Tuch bedarf offensichtlich keiner dichten Umhüllung, das Taschentuch kann genauso wie ein herkömmliches Papiertaschentuch verpackt werden.

Entsprechend kann man ein analog aufgebautes ölimprägniertes Toilettenpapier normal auf eine Rolle aufwickeln; ein dichter Behälter ist hierfür nicht notwendig.

Füllt man in die Mikrokapseln 14 einen Duftstoff ein, so erhält man ein Geruchsmuster, an welchem Kunden in einer Drogerie den Duftstoff in kleinsten Mengen dadurch ausprobieren können, daß sie das Muster auf der Haut reiben. Man kann so auch eine größere Anzahl unterschiedlicher Grundmuster unabhängig voneinander testen.

In Figur 2 ist die Wand der Mikrokapsel 14 mit 16 bezeichnet, der in ihr enthaltene Wirkstoff, z.B. Öl, mit 18. Die Mikrokapsel ist durch einen Bindemittelklumpen 20 mit Fasern 12 der Grundstruktur fest verbunden. Man erkennt, daß auf diese Weise am Randbereich der Verbindungsstelle große Aufreißkräfte erzeugt werden, wenn eine parallel zur Außenseite der Grundstruktur 10 gerichtete Kraft ausgeübt wird, wie dies beim Reiben des Tuches auf Haut der Fall ist.

Bei der abgewandelten Mikrokapsel 14 nach Figur 3 sind in die Wand 16 scharfkantige Partikel 22 eingebettet, die einerseits die Reibung zwischen der Mikrokapsel und der Haut vergrößern und zugleich Sollbruchstellen in der Kapselwand darstellen.

Bei der weiter abgewandelten Mikrokapsel 14 nach Figur 4 sind in das Gelmaterial der Kapselwand Gel-Anteile eingemischt worden, die sich mit dem Grundmaterial nicht homogen vermischen und unter Bildung innerer Grenzflächen getrennt hart werden. Beim Trocknen der so erhaltenen Wandabschnitte erhält man Sollbruchstellen am Rand dieser Wandabschnitte. Je nachdem, welches Fremdmaterial eingebaut wird, kann sich das Material der Wandabschnitte auch stärker (Wandabschnitt 24) oder weniger (Wandabschnitt 26) beim Trocknen zusammenziehen als das Wand-Grundmaterial, so daß die Außenfläche der Mikrokapsel eine zusätzliche Mikrostruktur erhält, die einerseits die Stärke der Verbindung zwischen Mikrokapsel und Grundstruktur über den Bindemittelklumpen 20 wegen besseren Verhakens des Bindemittels verbessert, andererseits wieder die Reibung zwischen Kapselaußenfläche und Haut vergrößert.

Es versteht sich, daß ein Wirkstoff-imprägniertes Tuch, wie es oben beschrieben wurde, auch andere Verwendungen zuläßt: z.B. schmiermittel-imprägnierte Tücher zum Überziehen von hochgenau bearbeiteten Werkstücken mit einem dünnen Schmierfilm; Schuhputztücher; Tücher mit Pfefferminzöl-Imprägnierung; Silberputztücher usw..

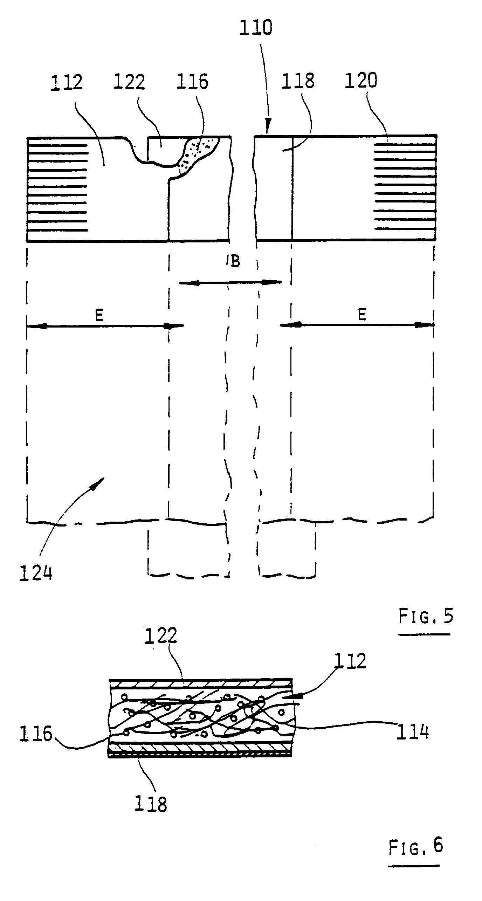

Ein in der Figur 5 insgesamt mit 110 bezeichneter Schal hat eine aus Vliesstoff hergestellte Kernschicht 112. Der Vliesstoff besteht seinerseits aus Zellstoff-Fasern 114, denen kleinere Anteile von Kunststoff-Fasern zugemischt sind. Derartige Vliesstoffe sind an sich bekannt.

In die lockere Kernschicht 112 sind zwischen und an den einzelnen Fasern 114 Mikrokapseln 116 angeordnet, z.B. unter Verwendung eines wasserlöslichen Bindemittels angeheftet. Dieser Einbau kann durch Aufsprühen einer entsprechenden Emulsion, durch Tauchen oder durch Aufstäuben der Mikrokapseln erfolgen.

Die Mikrokapseln 116 haben jeweils ein Wandmaterial, welches durch Körperwärme und/oder Schweißeinwirkung zersetzbar ist. Im Inneren der Mikrokapseln befindet sich ein Medikament, z.B. ein durch Körperwärme verdampfbares ätherisches Öl wie Pfefferminzöl, gegebenenfalls angereichert mit flüchtigen synthetischen Wirkstoffen gegen Erkältungskrankheiten.

Die Medikamentenimprägnierung, welche man durch die in der Kernschicht 112 enthaltenen Mikrokapseln 116 erhält, erstreckt sich nur über einen mittleren Bereich B des Schales 110, dessen Länge etwa 10 - 20 cm größer ist als der mittlere Umfang eines Halses, so daß die Medikamentenimprägnierung auch ohne exakte Positionierung des Schales 110 mit Sicherheit einmal um den Hals des Benutzers herumreicht. Typischerweise kann somit der imprägnierte mittlere Bereich B eine Länge von 60 cm aufweisen.

Eine Sperrschicht 118 überdeckt den imprägnierten Bereich B auf der Außenseite des Schals. Die Sperrschicht 118 kann typischerweise durch eine transparente dünne Kunststofffolie gebildet sein, die auf der einen Seite metallisiert ist, z.B. bedampft ist. Falls gewünscht, kann sich die Sperrschicht 118 aus ästhetischen Gründen auch über die gesamte Länge des Schales hin erstrecken. Umgekehrt kann die Sperrschicht 118 auch eine transparente Kunststoffschicht sein, und man kann die Außenseite der Kernschicht 112 durch Bedrucken verzieren, insbesondere im Bereich der beiden Schal-Endabschnitte E, welche sich an den imprägnierten mittigen Bereich B beidseits anschließen und typischerweise eine Länge von 40-80 cm haben können. Andere Arten der Verzierung der Endabschnitte E können in mechanischen Verformungen bestehen, z.B. Wellungen, Kräuselungen, ausgestanzten Öffnungen oder Fransen, wie sie bei 120 angedeutet sind.

Man erhält so insgesamt einen Schal 110 mit einer ansprechenden Außenseite.

Auf der Körperseite ist zumindest der imprägnierte Bereich B der Kernschicht 112 mit einer Deckschicht 122 versehen, die feuchtigkeitsdurchlässig ist, jedoch selbst keine Feuchtigkeit speichert. Auf diese Weise wird die Haut des Benutzers trocken gehalten, selbst wenn die Fasern 114 der Kernschicht 112 nach längerem Tragen des Schales 110 Feuchtigkeit aufgesogen haben.

Der oben beschriebene Schal 110 ist von einem Endlosmaterial 124 abgeschnitten, welches in Figur 5 gestrichelt angedeutet ist. Dieses Verbund-Endlosmaterial läßt sich einfach in einem kontinuierlichen Prozess aus preisgünstigen Bestandteilen herstellen.

Aus der obigen Beschreibung des Schals 110 ist ferner ersichtlich, daß dieser nur geringe Dicke zu haben braucht und somit einfach anstelle eines normalen Schales getragen werden kann. Nach Gebrauch wird der Schal 110 weggeworfen; falls gewünscht, kann er nach Erschöpfung der Medikamentenimprägnierung aber auch als reiner Schmuckschal weiter getragen werden.

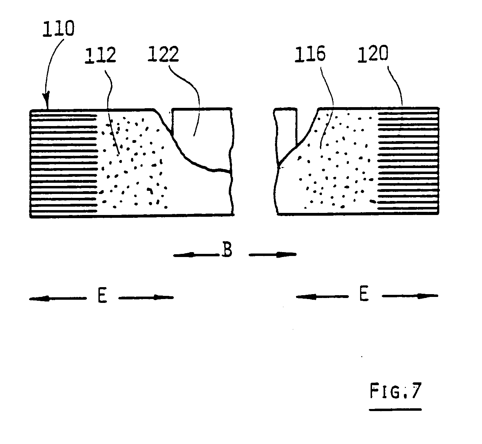

Beim Ausführungsbeispiel nach Figur 7 sind Teile des Schals, die obenstehend unter Bezugnahme auf die Figuren 5 und 6 schon in funktionsäquivalenter Form erläutert wurden, wieder mit denselben Bezugszeichen versehen. Es bestehen folgende Unterschiede:

Die ein flüchtiges Heuschnupfenmittel enthaltenden Mikrokapseln 116 sind in den Endbereichen E des Schals 110 vorgesehen und haben ein Wandmaterial, welches mechanisch durch Druck oder Reiben zerstörbar ist. Der um den Hals liegende Bereich B ist aus Ersparnisgründen von Medikament-Mikrokapseln 116 frei, nachdem diese dort vom Benutzer nur schwerer und mit kleinerer Effektivität zerstört werden können.

Ferner ist die Sperrschicht 118 weggelassen.

Es versteht sich, daß man die Ausführungsbeispiele nach den Figuren 5 und 7 auch kombinieren kann und die Medikament-Imprägnierung auf der Körperseite und der Außenseite des Schals auch über die gesamte Längserstreckung vorsehen kann, wenn hiergegen keine Kostengründen sprechen.

Auch kann man beim Ausführungsbeispiel nach Figur 7 anstelle von ein Medikament enthaltenden Mikrokapseln auch einen Duftstoff enthaltende Mikrokapseln verwenden.

Allen Ausführungsbeispielen ist gemeinsam, daß der imprägnierte Schal in seinen Abmessungen, insbesondere seiner Dicke einem normalen Baumwoll-, Seiden- oder Wollschal vergleichbar ist, da lockere Vliesstoffe aus saugfähigen Fasern zur Verfügung stehen, die auch bei geringer Dicke ein ausreichendes Aufnahmevermögen für Mikrokapseln und ein ausreichendes Saugvermögen für Schweiß aufweisen.

Der Schal läßt sich wegen seiner geringen Dicke auch praktisch faltenfrei umlegen, so daß eine gute Flächenberührung zwischen der Körperseite des Schals und einer dort vorgesehenen Medikament-Imprägnierung und der Haut gewährleistet ist.

Für den obenstehend unter Bezugnahme auf die Figuren 5 bis 7 beschriebenen, mit einer Medikamentimprägnierung versehenen Schal ist es wünschenswert, wenn die Wirkstoffabgabe nach Anlegen des Schales rasch die gewünschte Rate erreicht und anschließend konstant bleibt, bis der Wirkstoff erschöpft ist. Den zeitlichen Ablauf der Wirkstoffabgabe kann man über die Geometrie der Wirkstoffkapseln und auch über das Wandmaterial beeinflussen. Durch eine Mischung von Mikrokapseln unterschiedlicher Beständigkeit gegen Wärme und/oder Feuchtigkeit, kann man so den gewünschten zeitlichen Ablauf der Wirkstoffabgabe einstellen. Aus Gründen der einfacheren Herstellung wird es bevorzugt, diese Steuerung nur über die Kapselgeometrie vorzunehmen und möglichst mit einem einzigen Wandmaterial auszukommen. Hierbei werden folgende Faktoren in Betracht gezogen:

Bei gleicher Wandstärke sind große Mikrokapseln weniger beständig als kleine Mikrokapseln. Das Zerstören einer großen Mikrokapsel führt auch zur Freisetzung einer großen Wirkstoffmenge.

Bei gegebenem Kapseldurchmesser sind Kapseln mit großer Wandstärke stabiler als solche mit kleiner Wandstärke.

In einer Mischung aus Mikrokapseln sorgen somit leicht zerstörbare Mikrokapseln (z.B. dünnwandige, insbes. mit großen Durchmesser) für ein rasches anfängliches Freisetzen von Wirkstoff, Kapseln mittlerer Beständigkeit (mittlerer Durchmesser, ggf. mit etwas erhöhter Wandstärke) für die Wirkstoffabgabe in der Mitte des insgesamt angestrebten Anwendungszeitraumes (typischerweise für einen medizinischen Schal etwa 48 Stunden), und Mikrokapseln großer Beständigkeit (z.B. mit vergrößerter Wandstärke) für die Wirkstoffabgabe zu Ende des angestrebten Anwendungszeitraumes.

Ein praktisches Ausführungsbeispiel für eine mikroverkapselte Medikamentimprägnierung für einen medizinischen Schal, wie er anhand der Figuren 5 bis 9 beschrieben wurde, kann folgendermaßen aussehen:

Gelatine-Wandmaterial (Lösung 6,67 %): Bloomstandard 250 g Bloom; Standardviskosität (bei 60°C) 4,0 mPas; pH (bei 45°C) 5,9; isoelektrischer Punkt 4,9; Asche 0,7 %; Trockungsverlust 5,3 %; SO₂ 16 mg/kg; As weniger als 0,8 mg/kg; Schwermetalle weniger als 50 mg/kg.

Wirkstoff: 70 % Minzöl DAB 9, 30 % Fichtennadelöl DAB 9 (DAB = Deutsches Arzneibuch).

Größenverteilung der Mikrokapseln:

| Kapseldurchmesser | Wandstärke | Gewichtsprozent |
|---|---|---|
| 1 - 40 µm | ca. 2 µm | 20 % |
| 40 - 60 µm | ca. 5 µm | 30 % |
| 60 - 150 µm | ca. 5 µm | 35 % |
| 150 - 700 µm | ca. 15 µm | 7 % |
| 700 - 900 µm | ca. 10 µm | 5 % |
| 700 - 900 µm | ca. 40 µm | 3 % |

Die zusammen mit dieser Medikamentimprägnierung verwendete Vliesstoff-Kernschicht bestehen aus Viskosefasern, die dem US-Code of Federal Regulations 21, Food and Drugs § 176.170, § 177.2260 sowie § 177.2800 (1987) entsprechen. Das Vlies enthält ferner Polypropylenfasern, die ebenfalls den vorgenannten Gesetzesbedingungen entsprechen.

Das verwendete wässrige Bindemittel zum Anheften der Mikrokapseln an den Vliesstoff ist restliche Gelatine, die sich noch in der Verkapselungslösung befindet. Damit erfüllt das Bindemittel automatisch mit die gesetzlichen Bestimmungen, man braucht für dieses auch keine gesonderte Eingangs-Materialkontrolle. Auch ist so eine gutes Haften des Bindemittels am Kapsel-Wandmaterial gewährleistet. Das Auftragen der Bindemittel/Mikrokapsel-Mischung erfolgt durch schonendes Auftragen der Kapselmasse auf den Vliesstoff mit einer Rolle.

Der vorstehend beschriebene spezielle Menthol/Fichtennadel-Schal gibt seine Wirkstoffe innerhalb einer Tragzeit von etwa 48 Stunden ab.

## Patentansprüche

1. Wirkstoff-imprägniertes Tuch mit einer textilen Grundstruktur (112) und mindestens einer auf dieser angeordneten mikroverkapselten Medikamentenimprägnierung (116), deren Mikrokapseln (14) wenig stabil sind, wobei die Grundstruktur (112) die Form eines Schales hat und wobei das mikroverkapselte Medikament einen flüchtigen Wirkstoff z.B. ein flüchtiges ätherisches Öl wie Pfefferminzöl enthält, dadurch gekennzeichnet, daß die Wände (16) der Mikrokapseln (14) mindestens einer der Medikamentenimprägnierungen (116) gegenüber Wärme und/oder Feuchtigkeit wenig stabil sind und daß zumindest die äußersten Bereiche der Grundstruktur (10), aus den Wirkstoff nicht aufsaugenden Fasern (12) bestehen.

2. Tuch nach Anspruch 1, dadurch gekennzeichnet, daß eine der Medikamentimprägnierungen (116), die durch Wärme und/oder Feuchtigkeit aufbrechbare Mikrokapseln hat, nur über einen mittigen Bereich (B) der Längserstreckung vorgesehen ist, während Endbereiche (E) von dieser Imprägnierung frei sind.

3. Tuch nach Anspruch 1 oder 2, dadurch gekennzeichnet, daß eine der Medikamentenimprägnierungen (116) mit durch Druck oder Reibung aufbrechbaren Mikrokapseln nur in Endbereichen (E) der Längserstreckung vorgesehen ist.

4. Tuch nach einem der Ansprüche 1 - 3, dadurch gekennzeichnet, daß die Grundschicht (112) auf der Körperseite zumindest in einem mittleren Bereich (B) der Längserstreckung eine flüssigkeitsdurchlässige Deckschicht (122) trägt.

5. Tuch nach einem der Ansprüche 1 - 4, dadurch gekennzeichnet, daß die Grundschicht (112) auf ihrer Außenseite zumindest in einem mittigen Bereich (B) mit Medikamentenimprägnierung (116) eine gegen Feuchtigkeit und/oder Wärme undurchlässige Sperrschicht (118) trägt.

6. Tuch nach Anspruch 5, dadurch gekennzeichnet, daß die Sperrschicht (118) eine metallisierte Kunststofffolie ist.

7. Tuch nach einem der Ansprüche 1 - 6, dadurch gekennzeichnet, daß die Grundschicht (112) auf ihrer Außenseite und/oder im Bereich nicht imprägnierter Abschnitte (E) der Körperseite zumindest teilweise mit einer Verzierung, z.B. Fransen (20), einer Wellung, Kräuselungen, einer Bedruckung oder einem Lochmuster versehen ist.

8. Tuch nach einem der Ansprüche 1 - 7, dadurch gekennzeichnet, daß die Mikrokapseln (14) ein Gemisch aus Mikrokapseln unterschiedlichen Durchmessers sind.

9. Tuch nach Anspruch 8, dadurch gekennzeichnet, daß etwa die Hälfte der Mikrokapseln (116) einen Durchmesser <60 µm und der Rest der Mikrokapseln einen Durchmesser von >60 µm aufweist.

10. Tuch nach Anspruch 9, dadurch gekennzeichnet, daß etwa 20% der Mikrokapseln einen Durchmesser von <40 µm und etwa 30 % der Mikrokapseln einen Durchmesser von 40 bis 60 µm aufweisen.

11. Tuch nach Anspruch 9 oder 10, dadurch gekennzeichnet, daß etwa 35% der Mikrokapseln einen Durchmesser von etwa 60 bis 150 µm, etwa 7 % der Mikrokapseln einen Durchmesser von etwa 150 bis 700 µm und etwa 8 % der Mikrokapseln einen Durchmesser von etwa 700 bis 900 µm aufweisen.

12. Tuch nach einem der Ansprüche 1 bis 11, dadurch gekennzeichnet, daß die Mikrokapseln (14) ein Gemisch aus Mikrokapseln unterschiedlicher Wandstärke sind.

13. Tuch nach Anspruch 12, dadurch gekennzeichnet, daß das Verhältnis zwischen Wandstärke und Radius der Mikrokapseln zwischen etwa 0,02 und etwa 0,25 liegt, vorzugsweise bei etwa 0,1 liegt.

14. Tuch nach einem der Ansprüche 1 bis 13, dadurch gekennzeichnet, daß der Durchmesser der Mikrokapseln 10 bis 500 µm und die Dicke ihrer Wand etwa 1 bis 10 µm, vorzugsweise etwa 1 bis 5 µm beträgt.

15. Tuch nach einem der Ansprüche 1 bis 14, dadurch gekennzeichnet, daß in die Wand (16) der Mikrokapseln ein Fremdmaterial eingelagert ist, z.B. ein abweichende mechanische Eigenschaften aufweisendes, nicht mit dem Wandmaterial vermischbares Gelmaterial (24; 26) oder scharfkantige Partikel (22).

## Claims

1. Cloth impregnated with active substance, having a textile base structure (112) and disposed thereon at least one microencapsulated medicament impregnation (116), the microcapsules (14) of which have little stability, the base structure (112) being in the form of a scarf and the microencapsulated medicament containing a volatile active substance, for example, a volatile essential oil such as peppermint oil, characterised in that the walls (16) of the microcapsules (14) of at least one of the medicament impregnations (116) have little stability in respect of heat and/or moisture and that at least the outermost regions of the base structure (10) consist of fibres (12) that do not absorb the active substance.

2. Cloth according to claim 1, characterised in that one of the medicament impregnations (116), which has microcapsules burstable by heat and/or moisture, is provided only over a central region (B) of the longitudinal extent, whilst end regions (E) are free of this impregnation.

3. Cloth according to claim 1 or 2, characterised in that one of the medicament impregnations (116) having microcapsules burstable by pressure or friction is provided only in end regions (E) of the longitudinal extent.

4. Cloth according to any one of claims 1 to 3, characterised in that the base layer (112) on the body side carries a liquid-permeable covering layer (122) at least in a middle region (B) of the longitudinal extent.

5. Cloth according to any one of claims 1 to 4, characterised in that, at least in a middle region (B) having medicament impregnation (116), the base layer (112) carries on its outside a barrier layer (118) impermeable to moisture and/or heat.

6. Cloth according claim 5, characterised in that the barrier layer (118) is a metallised plastics film.

7. Cloth according to any one of claims 1 to 6, characterised in that, on its outside and/or in the region of non-impregnated sections (E) of the body side, the base layer (112) is provided at least in parts with a decoration, e.g. fringing (20), an undulation, ruffling, printing or an open-work pattern.

8. Cloth according to any one of claims 1 to 7, characterised in that the microcapsules (14) are a mixture of microcapsules of different diameters.

9. Cloth according to claim 8, characterised in that approximately half of the microcapsules (116) have a diameter of < 60 µm and the remainder of the microcapsules have a diameter of > 60 µm.

10. Cloth according to claim 9, characterised in that approximately 20% of the microcapsules have a diameter of < 40 µm and approximately 30% of the microcapsules have a diameter of 40 to 60 µm.

11. Cloth according to claim 9 or 10, characterised in that approximately 35% of the microcapsules have a diameter of approximately 60 to 150 µ, approximately 7% of the microcapsules have a diameter of approximately 150 to 700 µm and approximately 8% of the microcapsules have a diameter of approximately 700 to 900 µm.

12. Cloth according to any one of claims 1 to 11, characterised in that the microcapsules (14) are a mixture of microcapsules of different wall thickness.

13. Cloth according to claim 12, characterised in that the ratio between wall thickness and radius of the microcapsules lies between approximately 0.02 and approximately 0.25, preferably at approximately 0.1.

14. Cloth according to any one of claims 1 to 13, characterised in that the diameter of the microcapsules is 10 to 500 µm and the thickness of their wall is approximately 1 to 10 µ, preferably approximately 1 to 5 µm.

15. Cloth according to any one of claims 1 to 14, characterised in that incorporated in the wall (16) of the microcapsules is a foreign material, for example, a gel material (24; 26) immiscible with the wall material and having differing mechanical properties, or sharp-edged particles (22).

## Revendications

1. Chiffon imprégné d'une substance active, avec une structure de base (112) textile et au moins une imprégnation médicamenteuse (116) micro-encapsulée disposée sur cette structure, dont les micro-capsules (14) sont peu stables, la structure de base (112) ayant la forme d'une coquille et le médicament micro-encapsulé contenant un principe actif volatil, par exemple une huile éthérique volatile, telle que de l'huile de menthe, caractérisé en ce que les parois (16) des micro-capsules (14) d'au moins l'une des imprégnations médicamenteuses (116) sont peu stables face à la chaleur et/ou à l'humidité, et en ce qu'au moins les zones extérieures de la structure de base (10) sont constituées de fibres (12) n'aspirant pas le principe actif.

2. Chiffon selon la revendication 1, caractérisé en ce que l'une des imprégnations médicamenteuses (116), comportant des micro-capsules pouvant se rompre sous l'effet de la chaleur et/ou de l'humidité, n'est prévue que sur une zone centrale (B) de la longueur, tandis que les zones d'extrémité (E) sont exemptes de cette imprégnation.

3. Chiffon selon la revendication 1 ou 2, caractérisé en ce que l'une des imprégnations médicamenteuses (116) est prévue avec des micro-capsules pouvant se rompre sous l'effet de la pression ou du frottement, placées seulement dans des zones d'extrémité (E) de la longueur.

4. Chiffon selon l'une des revendications 1 à 3, caractérisé en ce que la couche de base (112) porte, sur la face destinée à être tournée vers le corps, au moins dans une zone centrale (B) de la longueur, une couche de couverture (122) perméable à un liquide.

5. Chiffon selon l'une des revendications 1 à 4, caractérisé en ce que la couche de base (112) porte sur sa face extérieure, au moins dans une zone centrale (B) ayant une imprégnation médicamenteuse (116), une couche de barrage (118) imperméable à l'humidité et/ou à la chaleur.

6. Chiffon selon la revendication 5, caractérisé en ce que la couche de barrage (118) est constituée d'une feuille en matière synthétique métallisée.

7. Chiffon selon l'une des revendications 1 à 6, caractérisé en ce que la couche de base (112) est pourvue, sur sa face extérieure et/ou dans la zone des sections (E) non imprégnées de la face tournée vers le corps, au moins partiellement d'une décoration, par exemple constituée par des franges (20), une ondulation, des fronçages, une impression ou bien un motif perforé.

8. Chiffon selon l'une des revendications 1 à 7, caractérisé en ce que les micro-capsules (14) sont un mélange de micro-capsules ayant des diamètres différents.

9. Chiffon selon la revendication 8, caractérisé en ce qu'à peu près la moitié des micro-capsules (116) ont un diamètre < 60 µm et le reste des micro-capsules ont un diamètre > 60 µm.

10. Chiffon selon la revendication 9, caractérisé en ce qu'à peu prés 20 % des micro-capsules ont un diamètre < 40 µm et à peu près 30 % des micro-capsules ont un diamètre compris dans la plage allant de 40 à 60 µm.

11. Chiffon selon la revendication 9 ou 10, caractérisé en ce qu'environ 35 % des micro-capsules ont un diamètre d'environ 60 à 150 µm, environ 7 % des micro-capsules ont un diamètre compris dans la plage allant d'environ 150 à 700 µm et environ 8 % des micro-capsules ont un diamètre compris dans la plage allant d'environ 700 à 900 µm.

12. Chiffon selon l'une des revendications 1 à 11, caractérisé en ce que les micro-capsules (14) sont un mélange constitué de micro-capsules ayant chacune des épaisseurs de paroi différentes.

13. Chiffon selon la revendication 12, caractérisé en ce que le rapport entre l'épaisseur de la paroi et le rayon des micro-capsules est compris dans la plage allant d'environ 0,02 à environ 0,25, de préférence est d'environ 0,1.

14. Chiffon selon l'une des revendications 1 à 13, caractérisé en ce que le diamètre des micro-capsules est compris dans la plage allant de 10 à 500 µm, et l'épaisseur de leur paroi est comprise dans la plage allant d'environ 1 à 10 µm, de préférence d'environ 1 à 5 µm.

15. Chiffon selon l'une des revendications 1 à 14, caractérisé en ce que dans la paroi (16) des micro-capsules est noyé un matériau étranger, par exemple un matériau se présentant sous forme de gel (24; 26), ou bien des particules (22) présentant des arêtes vives, matériau présentant des propriétés mécaniques différentes et qui n'est pas susceptible d'être mélangé avec le matériau de la paroi.
